# EUROPEAN PATENT SPECIFICATION

(11) **EP 1 464 697 B1**
(45) Date of publication and mention of the grant of the patent: **08.06.2011**
(21) Application number: 02716089.4
(22) Date of filing: 09.01.2002
(51) Int. Cl.: C12N 15/07, A61K 38/18, A61K 38/36

(54) **COMPOSITION for CREATING, REGENERATING AND REPAIRING TISSUES USING A CELL-CARRYING BIOLOGICAL IMPLANT WHICH IS ENRICHED WITH A PLATELET CONCENTRATE AND SUPPLEMENTS**
ZUSAMMENSETZUNG ZUR HERSTELLUNG, REGENERATION UND REPARATUR VON GEWEBEN UNTER VERWENDUNG EINES ZELLENTRAGENDEN BIOLOGISCHEN IMPLANTATS, DAS MIT EINEM BLUTPLÄTTCHENKONZENTRAT UND SUPPLEMENTEN ANGEREICHERT IST
COMPOSITION pour la CREATION, REGENERATION ET REPARATION DE TISSUS A L'AIDE D'UN IMPLANT BIOLOGIQUE PORTEUR DE CELLULES ET ENRICHI DE CONCENTRE DE PLAQUETTES ET DE SUPPLEMENTS

(43) Date of publication of application: 06.10.2004
(73) Proprietor: Gorrochategui Barrueta, Alberto, 48010 Bilbao Vizcaya (ES)
(72) Inventor: Gorrochategui Barrueta, Alberto, 48010 Bilbao (Vizcaya) (ES); Simon Elizundia, Josu, 48012 Bilbao (Vizcaya) (ES)
(74) Representative: ABG Patentes, S.L.
(86) International application number: PCT/ES2002/000007
(87) International publication number: WO 2003/057865

(56) References cited:
- WO-A-90/07931
- WO-A-92/09301
- US-A- 5 618 663

## Description

### OBJECT OF THE INVENTION

The present invention relates to a composition and procedure for tissue creation, regeneration and repair by a cell-bearing biological implant enriched with platelet concentrate and supplements, from among the compositions used for tissue regeneration which are derived from different conventional cell cultures originated from both heterologous and autologous cells.

The culture medium which nourishes the different cell lines includes high doses of various supplements in order to increase the number of cells obtained in the shortest possible period. These supplements are mainly nucleosides, hormones, cytosines, amino acids and vitamins, although also included are mineral salts, lipids and others.

The platelets added to the culture medium in which these high doses of supplements are added provide the platelet derived growth factors (PDGF) required to optimise the establishment, maintenance and extension of proliferation, so that the supernatant resulting from its activation may be used after adding calcium glucobionate to it. Additionally, these platelets together with the fibrinogen contained in them can be used as a support for cells derived from previously established cell cultures, or as constituents for the biological implant, biological glue and/or filler material.

In the present application document, the term "biological implant" refers to the platelet concentrate coagulated by the presence of: fibrinogen, fibronectin, calcium from the cell culture and ionised calcium from the platelets, ATP and ADP as activators of this activity, enriched with various other biological supplements and which will bear the cell cultures.

The term "biological glue" refers to the first few minutes of the biological implant and may or may not carry the established cell lines. A property of this biological glue is that it favours the healing process due to the growth factors included in it and which may act on the cells of the wound, inducing cell multiplication, differentiation or chemotaxis. Platelets are the only source of growth factor present in this biological glue.

"Filler material" relates to the newly formed thrombus in the culture medium which is later frozen and/or freeze-dried and/or pulverised in order to fill in osteoarticular defects.

### BACKGROUND OF THE INVENTION

The composition according to the invention obtains its cell culture from the cell culture conventionally employed in standard procedures for these types of cultures, complemented by high doses of supplements in order to improve the yield of the cell cultures and to improve the biological properties of this culture medium.

The cell cultures proceed from autologous or heterologous cells. They include chondrocytes, fibroblasts, osteoblasts, retinal pigment epithelium, hepatocytes and cells from the pilo-sebaceous unit among others, as well as embryonic cells.

Growth factors from platelet degranulation are used, among which are: in dense granules "serotonin, cathecolamines, ATP and ADP, calcium ions" and in alpha granules "albumin, beta-thromboglobulin, osteonectin, osteocalcin, platelet activation factor 4, platelet derived endothelial growth factor, epidermic growth factor" (Hudson-Goodmafl et al, 1990); "alpha plasmin inhibiting factor, fibrinogen, proacelerin, fibronectin, connective issue activation peptide III, transforming growth beta factor, insulin type growth factor" (Martin et al 1992); "high molecular mass quininogen, von Willebrand factor, thrombospondin, pospholipid, C1-sterease inhibitor, hepatocyte growth factor, platelet derived growth factors" (Miyazono and Takaku, 1989, Miyazono and Takaku 1989), (H.L. Wong and S.M. Wahl in "Peptide Growth Factors and their Receptors" Sporn Roberts Eds., Springer-Verlag. Berlin, p. 5 10, Terkeltraub and M. H. Grinsberg in "The Molecular and Cellular Biology of Wound Repair", Clark & Henson Eds., Plenum Press, New York, p. 38.)

Platelet extracts show a high mitogenous activity and have a known scarring effect. (D. M. Carter et al., "Growth Factors and Otehr Aspects in Wound Healing", Barbul. Pine, Cadwell Hunt Eds. Alan R. Liss Inc., New York 19998, pages 303 - 317, US Patent 4,760,131 and patent applications PCI WO 86/03 122, WO 88/03409 and WO 89/05656.

Exogenous proteins suh as thrombin are not added, nor are plasmin inhibitors such as alpha 2-antipamin or aprotinine; the presence of prothrombin and other coagulation factors in the biological glue of the invention can be used by activating either the extrinsic or intrinsic coagulation path. The preferred operation temperature is 37°C. In addition to these coagulable proteins, also not added is fibrinogen which as the former acts as a haemostatic material (see patent FR 2 448 900 by Immuno AF für Chemisch-Medizinische Produkte).

A remote antecedent of the state of the art would be Patent WO 90/07931 by Curatech, Inc., relating to recovery factors for pilous follicles using platelet growth factors which induce angiogenesis of blood origin and from either humans or other mammals, incorporating supplements as thrombin, adenosine diphosphate and collagen.

### DESCRIPTION OF THE INVENTION

The present invention relates to the culture medium obtained for the establishment, maintainance, propagation and freezing (with 10% DSMO) used in both human and animal cell lines of the examples described in this memory, with this culture medium characterised by having high concentrations of supplements and growth factors from the platelet concentrate from an either heterologous or autologous origin.

### A. Main composition and procedure thereof:

### A.1. Of the composition:

a) Cell culture medium used routinely in standard procedures of this type of culture, supplemented with a 10% platelet concentrate (the preferred variation is estimated at between 1 and 30%) stabilised during 24 hours in a culture oven. To this medium are added growth factors such as: IGF-I, IGF-II, TGF-b, although any other growth factors may be added which prove to aid cell proliferation.
b) To the cell culture are added the following supplements, meant to maintain the various cell lines: adenosine triphosphate (between 1 and 10 mg/l), sodium bicarbonate (between 1.2 and 5 g/l), cyticholine (histidine-5'-choline diphosphate (between 10 and 100 mg/l), ethanolamine (between 10 and 50 mg/l), phosphoethanolamine (between 5 and 25 µg/l), glucagon (between 1 and 5 mg/l), 1-glutamine (between 2 and 10 mM), sodium heparin (between 10,000 and 50,000 UI/l), hydrocortisone (between 10 and 100 mg/l), recombinant human insulin (between 100 and 1,000 UI/l), levothyroxin (between 50 and 200 mcg/l), linoleic acid (between 10 and 50 µg/l), oleic acid (between 10 and 50 mcg/l), sodium pyruvate (between 50 and 150 mg/l), sodium seleniate (between 10 and 50 mg/l), choleric toxin (between 0.1 and 1 mg/l), antibiotics (penicillin 100,000 UI/l, streptomycin 100 µg/l and amphoterycin B 2.5 µg/l), choline (between 1 and 30 mg/l), folic acid (between 1 and 10 mg/l), myo-inositol (between 1 and 40 mg/l), niacynamide (between 1 and 10 mg/l), p-amino benzoic acid (between 1 and 10 mg/l), D-pantotenic acid (between 1 and 15 mg/l), pyridoxine (between 1 and 10 mg/l), riboflavin (between 2 and 20 mg/l), thiamine (between 1 and 5 mg/l), vitamin B12 (between 1 and 10 µg/l) and amino acids {1-histidine (between 1 and 20 mg/l)}, {1-isoleukin (between 4 and 50 mg/1)}, {1-methynonine (between 1 and 25 mg/l)}, {1-phenyl alanin (between 2 and 20 mg/l)}, {1-triptophane (between 1 and 5 mg/l)}, {1-tyrosine (between 2 and 10 mg/l)} and optionally human albumin (between 100 and 1,000 mg/l) and human transferin (between 10 and 50 mg/l).
c) Platelet concentrate.

### A2. Procedure

The culture medium was evaluated according to the standard "Biological evaluation of medical devices, Part 5: Test for cytotoxicity: in vitro methods" (ISO 10993-5: 1992). The test was performed on cells 3T3 A31, which is a cell line from the albino Swiss mouse (ATCC CRL 1658). The cell culture is performed in culture flasks in a suitable environment for cellular proliferation.

The platelets used in this procedure were obtained in the following manner:

### A.2.1 With extraction by venous puncture of the patient:

Using 5 millilitre tubes containing 10% trisodium citrate as anticoagulant. The tubes were centrifuged at 200 g for 10 minutes. The blood was separated into its three components: plasma at the top, white corpuscles as a narrow line in the middle and erythrocytes on the bottom. Approximately 80% of the plasma layer volume was removed, with the remaining 20% used due to its high platelet concentration for supplementing the culture medium. These platelets are activated by the presence of calcium in the culture medium, forming a thrombus which can be removed and which is known as the filler material. Said platelet activation is responsible for the release of platelet derived growth factors (PDGF's) into the medium.

### A.2.2 From the blood bank, according to the following protocol:

### A.2.2.1 Preparation of the platelet concentrate.

Platelet concentrates are prepared from whole blood using large refrigerated centrifuges. The high g forces generated by these machines separate the blood into liquid plasma and its various cellular components.

When preparing the platelet concentrates the centrifuging is performed at ambient temperature; however, for other blood components it is performed at beween 1 to 6 °C. it is important to balance the material on opposite sides of the centrifuge head, which is easily achieved using rubber discs of differnet weights. It is convenient to place a plastic bag around the blood container for its protection, as it may break. Ports and tubes connected to the bag must also be protected against breakage. The centrifuge must not be slowed down manually, as this will affect the contents of the blood bag. All safety instructions must be strictly followed.

The protocol described is that which is most frequently followed in blood baks to separate blood products. With the centrifuge at low speeds, platelet rich plasma (PRP) is obtained from the blood unit on the top and erythrocytes on the bottom. The PRP is squeezed into an adjacent satellite bag, leaving the erythoroicytes in the main bag.

The two connected bags are again centrifuged, ths time at a high speed, so that a pellet of aggregated platelets is obtained from the PRP. Approximately 200 ml of platelet poor plasma are eliminated and 50 ml are left with the platelet pellet.

These 200 ml of plasma can be used to manufacture recovered plasma, frozen fresh plasma (FFP) or added to erythrocytes to obtain modified whole blood. Simultanoeusly, the bag with the platelet pellet and the 50 ml of plasma is separated from the primary erythrocyte bag and left to settle for 1 our at ambient temperature, in order to favour platelet disaggregation.

The platelets are then placed in a mechanical rotor to gently re-suspend the platelet pellet. Most platelets in the unit of whole blood are present in this platelet concentrate. In order to keep the platelets in their optimum functinoal state they must be constantly shaken at room temperature.

### A.2.2.2 Preparation of the platelet concentrate from multiple donors.

Platelets from multiple donors are obtained from whole blood 6 or 8 hours after collection (depending on the bag manufacturer and the type of anticoagulant employed) by low speed centrifugation, providing a platelet-rich plasma.

This plasma is transferred to a satellite bag and then intensely centrifuged to form a pellet of platelet aggregate and a platelet-poor plasma. The plasma is elimiated with the exception of 50 ml to form a platelet concentrate. The remaining 200 ml of platelet-poor plasma can be later added to the red corpuscles to obtain modified whole blood, or be frozen to obtain FFP, or used as recovered plasma for later elaboration.

This platelet concentrate is left to settle for 1 hour at room temperature, thereby allowing the platelet pellet to disintegrate slowly. The platelets are later subjected to a gentle and constant shaking process at ambient temperature (20 to 24 °C) during a conservation period of up to 5 days. It is important to ensure that the platelets maintain a pH of 6 or higher to maintain their funcitonality and that there is a minimum dose of 5.5 x 10¹⁰ platelets in 75% of the units studied.

### A.2.3 Preparation of serum with high concentration of PDGF's.

The platelets obtained either by venous puncture of the patient or from a blood bank can be activated according to the following protocol: For example, 9 millilitres are collected of platelet concentrate of any origin and between 1 and 2 millilitres are of calcium glucobionate are added (1 millilitre provides 4.5 mg of calcium element); this solution is taken to 37°C providing a serum rich in platelet-derived growth factors (PDGF's) and a thrombus which can be used as the filler material. This PDGF-rich serum can be used as an addition to the cell culture media or as a solution on cutaneous, osteoarticular or other defects in order to accelerate the wound healing processes. The serum can also be frozen and/or freeze-dried to extend its conservation until the time of use.

### A.3 Other specific supplements

In addition to the main composition and for cell lines other than retinal pigment epithelial type cells, fibroblasts, hepatocytes, tumoral lines and their equivalents, the following additions are defined with some or all of the following extra supplements: biotin (between 1 and 10 mg/l), dexametasone (between 1 and 10 mg/l), basic fibroblast growth factor (bFGF) (between 10 and 1,000 µg/l), teophilin (between 1 and 100 mg/l), 1-ascorbic acid (between 20 and 100 mg/l), human recombinant calcitonin (between 100 and 10,000 UI/l), calcitriol (between 0.1 and 10 µg/l), dexametasone (between 1 and 10 mg/l), inorganic salts such as monobasic anhydrous potassium phosphate (between 100 and 500 mg/l) and dibasic anhydrous potassium phosphate (between 1,000 and 2,500 mg/l) or other equivalent salts, human recombinant leukaemia inhibiting factor (100 to 10,000 UI/ml), thymidine (between 5 and 10 mg/l), guanosine (between 10 and 50 mg/l), cytidine (between 10 and 50 mg/l), uridine (between 10 and 50 mg/l), 2-b-mercaptoethanol (between 10 and 100 µg/l), forscholine (between 0.1 and 10 mg/l), 2-b-mercaptoethanol (between 10 and 100 mcg/l), adenosine triphosphate (between 1 and 10 mg/l) and human recombinant leukaemia inhibiting factor (100 to 10,000 UI/ml).

### B. Other specific compositions and procedures thereof:

These specific complements are incorporated as follows:

### B.1 Of the composition

- For adipocytes: biotin (between 1 and 10 mg/l), dexametasone (between 1 and 10 mg/l).
- For melanocytes: basic fibroblast growth factor (bFGF) (between 10 and 1,000 µg/l), teophilin (between 1 and 100 mg/l).
- For chondrocites and osteoblasts: 1-ascorbic acid (between 20 and 100 mg/l), human recombinant calcitonin (between 100 and 10,000 UI/l), calcitriol (between 0.1 and 10 µg/l), dexametasone (between 1 and 10 mg/l), inorganic salts such as monobasic anhydrous potassium phosphate (between 100 and 500 mg/l) and dibasic anhydrous potassium phosphate (between 1,000 and 2,500 mg/l or equivalent salts.
- For stem cells: human recombinant leukaemia inhibiting factor (100 to 10,000 UI/ml), nucleosides, adenosine triphosphate (between 1 and 10 mg/l), thymidine (between 5 and 10 mg/l), guanosine (between 10 and 50 mg/l), uridine (between 10 and 50 mg/l), 2-b-mercaptoethanol (between 10 and 100 µg/l), forscholine (between 0.1 and 10 mg/l.

### B.2 Culture and maintenance of these cell lines.

The culture medium was prepared with the above indicated components. Antibiotics were used in solution with the following final doses: penicillin, 100,000 UI/l, streptomycin 100 µg/l and amphoterycin B 2.5 µg/l. The pH was adjusted to 7.40 and the osmolarity for human cells of the culture was 290 mOsm/kg, assumed to be ideal for maintenance in vitro, whereas for other species such as mice the osmolarity of the initial cultures was 310 mOsm/kg.

The cells were kept in culture flasks of 25 cm² or 75 cm² in a routine culture medium with all the supplements and products of platelet degranulation, in an oven at 37°C with a 5% CO₂ atmosphere.

When the cells contained in a culture flask are adhered to its surface, forming a monolayer (exponential growth stage) the cells are obtained by the following process:
- Decanting the culture medium in the flask:
- Washing with a phosphate buffered saline (pH 7.3 at 4°C) and later decanting;
- Addition of EDTA-PBS (between 1 and 10 mM) and vigorous shaking, in order to release the cells adhered to the bottom of the flask;
- Addition of 3 ml of phosphate buffered saline to reach a total final amount or 10 ml and resuspension. Afterwards, centrifuging (Minifuge T Heraeus Christ) in a 50 ml conical tube at 200 g for 5 minutes at 37°C;
- Decanting the supernatant while the pellet is resuspended in a known volume of PBS, proceeding to perform a cellular viability study using the exclusino method with tripan blue at 0.1% in a Burker type corpuscle counting machine. If the number of viable cells is above 95% they are again resuspended in the required volume to obtain the correct cell concentration. The cell concentration used in this work was 5 x 10⁵ cells in 0.1 ml of PBS.

All of the above steps were performed in strictly sterile conditions obtained with Gelaire BSB4 steirile laminar flow chambers.

In order to detect possible contamination by mycoplasmas the cultures were periodically subjected to a fluorescence mycoplasma detection test according to the following protocol:
Cells maintained in the culture were fixed in Methanol - Acetic acid (3:1) and submerged in a Hoechst 33258 buffer solution (bisbenzimidazol) prepared from a sterile stock solution consisting of 100 ml of PBS and 15 mg of Hoechst 33258, with later dilution to 1:500 in PBS during 30 min in the dark and at ambient temperature. The cells were observed in a fluorescence microscope; in the event of contamination by mycoplasmas these are seen as small fluorescent bodies in the extrannuclear and intercellular space.

### B.3 Maintenance of the cell lines at low temperatures.

Long-term maintenance of a cell stock was obtained by freezing culture cells in liquid nitrogen. The method followed for this freezing was as follows:
When the culture cells begin their exponential growth stage the cell viability is calculated by dyeing with tripan blue. If it is greater than 95% they are resuspended in a solutin of the whole culture medium and DMSO (dimethylsulphoxide) at 10%.
The concentration is adjusted to 2x10⁶ cells/ml. This suspension is introduced in freezing blisters and submerged in liquid nitrogen. Afterwards they are stored in a freeze chamber at -180°C. Cells are thawed by heating the blisters in a bath at 37°C with a later centrifugation in order to eliminate the DMSO. The pellet is resuspended in whole culture medium and seeded in a culture flask, and in this manner introduced in an incubation oven at 37°C.

### B.4 Maintenance of the stem cells

After selecting the stem cells obtained from an embryo, foetus or adult by enzymatic process, tissue cell culture or ovocytes, the maintenance of these stem cells in the culture medium is conditinoed or not by STO cells, STO SNL 76/7 or VERO during 48 hours, and up to 40% of this conditioned medium used for cell cultures. It has been known to occasionally use STO SNL 76/7 cells (Mcmahon and Bradley, 1990) or VERO cells in the form of mitotically inactivated monolayers (Evans and Kauffman, 1981) used routinely in standard procedures for this type of cultures (Nagy et al., 1993). These monolayers can secrete embryotrophic substances such as TGF-a (transforming growth factor a), TGF-b (transforming growth factor b), PDGF-a (platelet-derived growth factor a) and IGF-I and II (insulin type growth factors), and thus support both embryo development and stem cell maintenance. It is also possible to provide human recombinant LIF (leukaemia inhibiting factor) in a dose between for example 100 and 10,000 units/ml (Kitamura et al., 1989) for maintaining the pluripotent phenotype of stem cells. In order to obtain levels which allow formation of stem cell niches which remain undifferentiated (Rathjen et al., 1990) this factor can on its own maintain and serve to isolate stem cells (Ernst et al., 1994; Nagy et al., 1993). Stem cells from the pilo-sebaceous unit ept in vitro present a directly provable alkaline phospphatase activity as in blastocytes (Johnson et al., 1977). This property allows the use of histochemical marking to detect stem cells, as well as expliciting the undifferentiated state of these cells (Piquet-Pellorce et al., 1994; WO90/08188; PCT AU 89/00330) by positive marking for alkaline phosphatase of the embryoid bodies and stem cells.

The cells empoyed may be pluripotent or totipotent cells from diverse origins, such as the bone marrow, but embryonic cells or differentiated cells from the same patient may also be used. The latter and those obtained from the bone marrow have an autologous origin and therefore do not generate an autoimmune response.

For this purpose, the cells are isolated by an enzymatic process with trypsin and/or dispase/collagenase depedning on the cell type. The cell lines are taken to a flask with 25 cm² surface area and the subcultures made when observing the presence of embryoid bodies or the disappearance of cells from the monolayer, maintaining them in the culture oven subjected to routine passages every 48 hours. Afterwards they can be introduced in the biological glue which will later become the biological implant.

Said biological implant can be left in the oven for over 90 days, with its cells being viable and allowing a new cellular passage as long as the culture conditions for each cell line are maintained.

Cells used in both the biological glue and in the biologicla implants are for example obtained in the following manner: the cell line is trypsinised, the trypsin is inactivated by an inactivator and centrifuged at 200g. The supernatant is removed and the pellet is resuspended in 1 ml of culture medium. An aliquot containing 10x10⁶ cells is prepared for a final volume of 10 ml, considering that 1 ml is the platelet concentrate. The entire procedure is performed at 37°C.

If used as biological glue, it presents excellent adhesion to tissues or prosthetic materials; if a biological implant is desired it is taken to the oven, where two variants are obtained: after retracting the 10 ml of biological glue in a test tube with an internal 10 mm light, after 24 hours a 10x1 mm cylinder is presented, in an experience performed thre times.

If this is used on culture flasks or multiplied from a culture in 6 dimples, retraction does not occur so that this proves to be the ideal medium in which to achieve a lamina.

Patches are obtained made of fibroblasts and keratinocytes superimposed over those which resemble the dermoepidermis. Isoleukin is the aminoacid required to maintain the growth of keratinocytes, although an increase in the other amino acids has positive effects on the proliferation potential of the different cell lines.

In the case of osteoblasts variable layers are produced in which calcification can be induced in varying degree, in order to cover or fil in bone defects. The extracellular mesh produced by the osteoblasts in cultures made in vitro presents a varying degree of mineralisation, so that it allows introduction in osteoarticular defect with the same cells (auto-grafting) or after eliminating these (allografting).

As a biological glue it can be used in cases of retinal detachment, even bearing pigment epithelium cells of said retina.

Platelet factors released into both the culturemedium and the inside of the biological glue or implant achieve and increase in cell proliferation and encourage growth, while simultaneously preventing the degradation of the product which adheres to the exposed surfaces.

The use of protease inhibitors such as epsilonaminocaproic acid (200 - 400 µg/ml of gel) and tranexamic acid (200 - 400 µg/ml of gel) was not considered necessary, and the addition of other types of protease (chicken egg yolk, from Sigma Co. type III) was discarded.

### C. Use of bioimplants.

Bioimplants can be used in the following manners:
a) The newly prepared biological glue can serve as a material for direct adhesion on the site of the wound, such as in cases of retinal detchment with cells proceeding directly from the cultures or with those conserved in liquid nitrogen. With this glue it is possible to fill in the osteoarticular defect, or at least it can be used as a filler material for a large extent of said defect.
b) When the biological implant is to be applied the wound or defect can be soaked in the biological glue and the implant later compressed at the site of the wound. Within 48 hours cell proliferation is observed and after 3 weeks calcification nodules can be observed if the cells are osteoblasts.
   As biological glue, given the speed with which the thrombus is produced the chondrocites are adhered to the wounded surfaces, as are other cell types such as fibroblasts implanted in the dermal or hypodermal region or pigment epithelium cells in the retina. This last case deserves special attention, as the final volume of the biological glue when the thrombus forms is almost negligible (1 mm³ per millilitre on the avegrage) and in view of the regenerative ppower of these cells (because of their pluripotent nature). The field of retinal regeneration would be pened in cases such as retinitis pigmentosa and retinal detachmnent.
   One of the main advantages of this procedure is the absence of an exogenous protein addition, the absence of animal serum, which reduces the risks of accidental contamination.
c) As filler material it is obvious that the frozen, desecated and/or freeze-dried product has a durability of more than three years. Both desecated products and the implant should be irrigated with the biological glue, and such solution should be applied on the wound to be treated.

The procedure of the invention is therefore particularly well suited for the current need to use autologous products.

These products: culture medium, biological glue, biological implant and filler material can be used in the treatment of traumatic or surgical wounds, in bone implants or osteoarticular reconstructions, in maintenance of grafts (skin and bone, among others) and in long-term in-vitro maintenance of biological implants. The same glue can be used to fix prosthetic pieces including bone prostheses, osteosunthesis pieces and dental prostheses, and to fix and provide cohesion to bone filler materials such as calcium carbonate, hydroxyapathite or biodegradable polymers such as poly(lactic acid).

As regards the industrialisation of the prodcut, the basic composition to add to the specific cell culture is embodied in a single supplement complex, prepared previously in order to facilitate and speed up the operations described in the present memory, as well as for direct use with cell lines that do not require extra supplements, leaving for each specific case the incorporation of the two optional supplements: human albumin and transferin.

The same is true for the extra supplements intended for cell lines which require their addition: for each specific cell line the mixture corresponding to the correct group of supplements is previosuly available, for the same purpose of facilitating the specific operations.

### PREFERRED EMBODIMENT OF THE INVENTION

### Basic composition.

It incorporates the following biological supplements: adenosine triphosphate (1 mg/l), aminoacids (1-histidine 2 mg/l; 1-isoleukin 4 mg/l; 1-methyonine 1 mg/l; 1-phenylalanine 2 mg/l; 1-triptophane 1 mg/l; 1-tyrosine 2mg/l), antibiotics (penicilln 100,000 UI/l, streptmycin 100 µg/l and amphoterycin B 2.5 µg/l), sodium bicarbonate (1.2 g/l) cyticholine (choline histidine-5'-diphosphate, 10 mg/l), ethanolamine 10 mg/l, phoshoethanolamine 5 mg/l, glucagon 1 mg/l, linoleic acid 10 µg/l, oleic acid 10 µg/l, 1-glutamine 2 mM, sodium heparin 10,000 UI/l, hydrocortisone 10 mg/l, recombinant human insulin 100 UI/l, levothyroxin 50 mg/l, sodium pyruvate 50 mg/l, selenious acid 10 µg/l, choleric toxin 0.1 mg/l and vitamins (d-biotin 1 mg/l, choline 1 mg/l, folic acid 1 mg/l, myo-inositol 1 mg/l, niacynamide 1 mg/l, p-amino benzoic acid 1 mg/l, D-pantotenic acid 1 mg/l, pyridoxine 1 mg/l, riboflavin 2 mg/l, thiamin 1 mg/l, vitamin B12 1 µg/l).

### Other specific supplements.

In certain cases, in addition to the optional human albumin 100 mg/l and transferin 10 mg/l, the following supplements are added: human recombinant calcitonin (1,000 UI/l) recombinant human leukaemia inhibitor factor (1,000 UI/ml), vitamins (D-biotin 1 mg/l, dexametasone 1 mg/l), basic fibroblast growth factor (b-FGF) (10 µg/l), teophilin (1 mg/l), 1-ascorbic acid (20 mg/l), calcitriol (0.5 µg/l), inorganic salts such as monobasic anhydrous potassium phosphate (200 mg/l) and dibasic anhydrous potassium phosphate (1,100 mg/l) or other equivalent salts, forscholine (0.1 mg/l), nucleosides; adenosine 10 mg/l, thymidine 5 mg/l, guanosine 10 mg/l, cytidine 10 mg/l, uridine 10 mg/l, 2-b-mercaptoethanol 10 mcg/l.

As relates to the examples shown below these are meant to further illustrate the model and represent the preferred embodiment. These examples were prepared and tested as follows:

### Example 1. Creation of a dermocutaneous substitute with a capacity to develop pilous follicles.

The cell lines are obtained from a skin punch of variable dimater or from the follicle unit or the pilous sebaceous unit. They are cut into three parts, depending on their widths, and from the epidermis are obtained keratinocytes and melanocytes, from the dermis the fibroblasts and from the hypodermis the adiposites which are maintained with the following supplemtns: vitamins (D-biotine 1 mg/l, dexametasone 1 mg/l), culturing each group separately. The culture medium used in this example corresponds in essence to that used for the stem cells.

Afterwards the adipocytes are obtained by enzymatic processing with dispase/collagenase. 10x10⁶ of these are resuspended in 5 ml of fresh culture with a 10% platelet concentrate, adding the specific supplements; it is seeded in a 75 cm² flask and left to coagulate in an oven for 1 hour. The fibroblasts are extracted in the same manner, and finally the keratinocytes which have been cultivated in their specific medium, that is: recombinant human leukaemia inhibition factor, 1,000 UI/ml, forscholine 0.1 mg/l to maintain the specificity of the cutaneous stem cells, with the same procedure; changes are then made with the enriched medium during 15 days in order to increase the thickness of the keratinocyte layer.

In this manner a cutaneous substitute is obtained which after a keratinisation process by exposure to air can be used in daily clinical practice.

### Example 2. Biological implant containing osteoblasts.

The cell line is obtained from the trabecular bone or from a bone crest or bone puncture. It is washed well to remove remains of bone marrow or periostium. The cells are obtained in the same manner as in example 1, observing the particular growth properties of this cell line and resuspending in 10 ml final of the culture medium and 10% platelt concentrate, as well as the following specific supplements: 1-ascorbic acid (20 mg/l), calcitriol (0.5 µg/l) recombinant human calcitonin (1,000 UI/l), inorganic salts such as monobasic anhydrous potassium phosphate (200 mg/l) and dibasic anhydrous potassium phosphate (1,100 mg/l) or other equivalent salts. They are taken to an oven to produce retraction of the platelet thrombus in a tube of 10 cm by 1 cm of inner diameter, leving a cylinder 10 mm in length and 1 mm in diameter.

### Example 3. Biological implant and/or glue containing chondrocites.

Obtaining the chondrocite culture from the articular cartilage by processing with dispase/collagenase.

Maintaining the cell line with the following specific supplements: 1-ascorbic acid (20 mg/l), calcitriol (0.5 µg/l), inorganic salts such as monobasic anhydrous potassium phosphate (200 mg/l) and dibasic anhydrous potassium phosphate (1,100 mg/l) or other equivalent salts and later recovery with similar enzymatic processing. 10x10⁶ cells/ml of culture medium are resuspended as in the previous cases. The damaged cartilage is reached by arthroscopic surgery, preparing the bed for the intervention and the defect is filled at the moment when the cell suspension and the biological glue is prepared. It coagulates quickly.

### Example 4. Biological glue for repairing retinal detachment and/or regeneration in the case of pigment retinitis.

Cells are cultivated from the retinal pigment epithelium, with a proven capacity for regeneration of the retina. The culture medium used is the standard medium. It is resuspended as in Example 3 and the detached area is filled in. It should be remarked that this medium occupies finally 1 cubic millimetre in the experiences performed in the laboratory.

These procedures, performed at the operating table, show the advantage of providing great amounts of platelet growth factor in the wound area as the platelets are activated after they are placed in contact with the culture medium.

### General procedure.

The development of the culture medium is supplemented by growth factor obtained from the platelet concentrate, of either autologous or heterologous origin.

It can routinely have a concentration of 1%. Other growth factors (IGF-I, IGF-II and TGF-b, among others) and supplements are added depending on the cell type to be kept in vitro. Maintenance of the stem cells requires recombinant human leukaemia inhibiting factor.

The biological implant is obtained from the activation of the platelet concentrate, its coagulation and retraction induced by the calcium present in the cell culture medium. The biological glue is formed in the first few minutes of platelet activation and is used as a filler in any type of wounds. The filler material is obtained from the thrombus created by the platelet retraction, in order to fill in osteoarticular defects.

### Specific procedure 1.

The pilosebaceous unit is meant for the routine maintenance in the culture of stem cells, among the generally obtained cells, of 12 pilosebaceous units for a culture flask with 25 cm² surface area, subcultures are made when the cellular subconfluence is reached and usign the specific culture medium each time. If the latter is not used the cells will lose capabilities and a skin will develop which can be used as a skin substitute.

### Specific procedure 2.

Osteoblasts are obtained from a cell culture. Osteoarticular defects of any known aetiology are cleaned and provided with a geometrical shape (cylindrical, cubic, or other) in accordance with the implant material to be used. These biological implants incorporate the culture osteoblasts; they can adhere much better by use of the biological glue prepared immediately before. The filling in can be performed with the frozen, freeze-dried or pulverised material. Small osteoarticular defects can be filled with biological glue prepared immediately before containing or not the previosuly established cell lines. In any case, the biological glue may or may not contain cells at the time of application. Any type of implant and/or dressing can be submerged and/or soaked in the biological glue as well as in the place of implantation or to be covered.

### Specific procedure 3.

In the case of retinal detachment or pigment retinitis the preferred cell line is cultured, such as retinal pigment epithelium. Culture cells are obtained, resuspended and the platelet concentrate added to form the biological glue with which the retinal defects are filled in.

### Specific procedure 4.

In osteotendinosal tears, the biological glue can be introduced directly with or withut cells, while for large defects implants may be used which substitute bone with the appropriate shape, coating it with biological glue or with the pulverised material.

### Speicific procedure 5.

In the case of melanocytes the procedure begins with the enzymatic processing with trypsin of 12 pilosebaceous units, obtaining cells from the dermoepidermal union, mainly consisting of melanocytes among other cells, and these cells are incorporated in a flask of 25 cm² surface area with the subcultures performed when the cellular subconfluence is attained, always using the specific culture medium to prevent the cells from losing capabilities and developing until the time of introduction in the wounded areas.

The various altenatives and modifications proposed shall be subject to the forms and variations encompassed herein.

## Claims

1. Composition for the establishment, maintenance, propagation and freezing of cell lines which comprises:
· platelet concentrate between 1 and 30%,
· growth factors such as IGF-1, IGF-11, TGF-b and,
· the following supplements:
- adenosine triphosphate between 1 and 10 mg/l,
- sodium bicarbonate between 1.2 and 5 g/l,
- cyticholine between 10 and 100 mg/l,
- ethanolamine between 10 and 50 mg/l,
- phosphoethanolamine between 5 and 25 µg/l,
- glucagon between 1 and 5 mg/l,
- 1-glutamine between 2 and 10 mM,
- sodium heparin between 10,000 and 50,000 UI/l,
- hydrocortisone between 10 and 100 mg/l,
- recombinant human insulin between 100 and 1,000 UI/l,
- levothyroxin between 50 and 200 µg/l,
- linoleic acid between 10 and 50 µg/l,
- oleic acid between 10 and 50 µg/l,
- sodium pyruvate between 50 and 150 mg/l,
- sodium seleniate between 10 and 50 mg/l,
- choleric toxin between 0.1 and 1 mg/l,
- antibiotics,
- choline between 1 and 30 mg/l,
- folic acid between 1 and 10 mg/l,
- myo-inositol between 1 and 40 mg/l,
- niacynamide between 1 and 10 mg/l,
- p-amino benzoic acid between 1 and 10 mg/l,
- D-pantotenic acid between 1 and 15 mg/l,
- pyridoxine between 1 and 10 mg/l,
- riboflavin between 2 and 20 mg/l,
- thiamine between 1 and 5 mg/l,
- vitamin B12 between 1 and 10 µg/l, and
- amino acids.

2. Composition according to claim 1, wherein the antibiotics are selected from the group consisting of:
- penicillin in a concentration of 100,000 UI/l,
- streptomycin in a concentration of 100 µg/l, and
- amphoterycin B in a concentration of 2.5 µg/l.

3. Composition according to claim 1, wherein the amino acids are selected from the group consisting of:
- 1-histidine between 1 and 20 mg/l,
- 1-isoleukin between 4 and 50 mg/l,
- 1-methynonine between 1 and 25 mg/l,
- 1-phenylalanine between 2 and 20 mg/l,
- 1-triptophane between 1 and 5 mg/l and
- 1-tyrosine between 2 and 10 mg/l.

4. Composition according to claim 1, that further comprises human albumin present in a concentration from 100 to 1,000 mg/l and human transferrin present in a concentration from 10 to 50 mg/l.

5. Composition according to claim 1, wherein the cell lines are both human and animal cell lines.

6. Composition according to claim 1, wherein the cells are selected from the group consisting of fibroblasts, hepatocytes, retinal pigment epithelial cells and tumoral lines cells.

7. Composition according to claim 1, wherein the composition is a specialized cell culture media adapted for the culture of adipocytes and further comprising:
- biotin in a concentration from 1 to 10 mg/l, and
- dexametasone in a concentration from 1 to 10 mg/l.

8. Composition according to claim 1, wherein the composition is a specialized cell culture media adapted for the culture of melanocytes and further comprising:
- basic fibroblast growth factor (bFGF) in a concentration from 10 to 1,000 µg/l, and
- teophilin in a concentration from 1 to 100 mg/l.

9. Composition according to claim 1, wherein the composition is a specialized cell culture media adapted for the culture of chondrocites and osteoblasts and further comprising:
- 1-ascorbic acid in a concentration from 20 to 100 mg/l,
- human recombinant calcitonin in a concentration from 100 to 10,000 UI/l,
- calcitriol in a concentration from 0.1 to 10 µg/l,
- dexametasone in a concentration from 1 to 10 mg/l and
- inorganic salts.

10. Composition according to claim 9, wherein the inorganic salts are selected from the group consisting of monobasic anhydrous potassium phosphate in a concentration from 100 to 500 mg/l and dibasic anhydrous potassium phosphate in a concentration from 1,000 to 2,500 mg/l.

11. Composition according to claim 1, wherein the composition is a specialized cell culture media adapted for the culture of stem cells and further comprising:
- human recombinant leukaemia inhibiting factor in a concentration from 100 to 10,000 UI/l,
- nucleosides,
- adenosine triphosphate in a concentration from 1 to 10 mg/l,
- thymidine in a concentration from 5 to 10 mg/l,
- guanosine in a concentration from 10 to 50 mg/l,
- uridine in a concentration from 10 to 50 mg/l,
- 2-b-mercaptoethanol in a concentration from 10 to 100 µg/l, and
- forscholine in a concentration from 0.1 to 10 mg/l.

12. Composition according to claim 1 that further comprises DMSO (dimethylsulphoxide) at 10% in order to conserve the cell viability during storage in low temperature.

## Patentansprüche

1. Zusammensetzung zum Etablieren, Erhalten, Vermehren und Einfrieren von Zelllinien, die umfasst:
• Blutplättchenkonzentrat zwischen 1 und 30%,
• Wachstumsfaktoren wie IGF-1, IGF-11, TGF-b und,
• die folgenden Ergänzungsmittel:
- Adenosintriphosphat zwischen 1 und 10 mg/l,
- Natriumbicarbonat zwischen 1,2 und 5 g/l,
- Cyticholin zwischen 10 und 100 mg/l,
- Ethanolamin zwischen 10 und 50 mg/l,
- Phosphoethanolamin zwischen 5 und 25 µg/l,
- Glucagon zwischen 1 und 5 mg/l,
- 1-Glutamin zwischen 2 und 10 mM,
- Natriumheparin zwischen 10.000 und 50.000 UI/l,
- Hydrocortison zwischen 10 und 100 mg/l,
- rekombinantes humanes Insulin zwischen 100 und 1.000 UI/l,
- Levothyroxin zwischen 50 und 200 µg/l,
- Linolsäure zwischen 10 und 50 µg/l,
- Ölsäure zwischen 10 und 50 µg/l,
- Natriumpyruvat zwischen 50 und 150 mg/l,
- Natriumseleniat zwischen 10 und 50 mg/l,
- Choleratoxin zwischen 0,1 und 1 mg/l,
- Antibiotika,
- Cholin zwischen 1 und 30 mg/l,
- Folsäure zwischen 1 und 10 mg/l,
- Myo-Inositol zwischen 1 und 40 mg/l,
- Niacynamid zwischen 1 und 10 mg/l,
- p-Aminobenzoesäure zwischen 1 und 10 mg/l,
- D-Pantotensäure zwischen 1 und 15 mg/l,
- Pyridoxin zwischen 1 und 10 mg/l,
- Riboflavin zwischen 2 und 20 mg/l,
- Thiamin zwischen 1 und 5 mg/l,
- Vitamin B12 zwischen 1 und 10 µg/l, und
- Aminosäuren.

2. Zusammensetzung nach Anspruch 1, wobei die Antibiotika ausgewählt sind aus der Gruppe bestehend aus:
- Penicillin in einer Konzentration von 100.000 UI/l,
- Streptomycin in einer Konzentration von 100 µg/l, und
- Amphoterycin B in einer Konzentration von 2,5 µg/l.

3. Zusammensetzung nach Anspruch 1, wobei die Aminosäuren ausgewählt sind aus der Gruppe bestehend aus:
- 1-Histidin zwischen 1 und 20 mg/l,
- 1-Isoleucin zwischen 4 und 50 mg/l,
- 1-Methionin zwischen 1 und 25 mg/l,
- 1-Phenylalanin zwischen 2 und 20 mg/l,
- 1-Tryptophan zwischen 1 und 5 mg/l und
- 1-Tyrosin zwischen 2 und 10 mg/l.

4. Zusammensetzung nach Anspruch 1, die ferner humanes Albumin, das in einer Konzentration von 100 bis 1.000 mg/l vorliegt, und humanes Transferrin, das in einer Konzentration von 10 bis 50 mg/l vorliegt, umfasst.

5. Zusammensetzung nach Anspruch 1, wobei die Zelllinien sowohl humane also auch tierische Zelllinien sind.

6. Zusammensetzung nach Anspruch 1, wobei die Zellen ausgewählt sind aus der Gruppe bestehend aus Fibroblasten, Hepatozyten, Netzhautpigmentepithelzellen und Tumorzelllinien.

7. Zusammensetzung nach Anspruch 1, wobei die Zusammensetzung ein spezialisiertes Zellkulturmedium ist, das angepasst ist zur Züchtung von Adipozyten und ferner umfasst:
- Biotin in einer Konzentration von 1 bis 10 mg/l, und
- Dexametason in einer Konzentration von 1 bis 10 mg/l.

8. Zusammensetzung nach Anspruch 1, wobei die Zusammensetzung ein spezialisiertes Zellkulturmedium ist, das angepasst ist zur Züchtung von Melanozyten und ferner umfasst:
- basischen Fibroblastenwachstumsfaktor (bFGF) in einer Konzentration von 10 bis 1.000 µg/l, und
- Teophilin in einer Konzentration von 1 bis 100 mg/l.

9. Zusammensetzung nach Anspruch 1, wobei die Zusammensetzung ein spezialisiertes Zellkulturmedium ist, das angepasst ist zur Züchtung von Chondrozyten und Osteoblasten und ferner umfasst:
- 1-Ascorbinsäure in einer Konzentration von 20 bis 100 mg/l,
- humanes rekombinantes Calcitonin in einer Konzentration von 100 bis 10.000 UI/l,
- Calcitriol in einer Konzentration von 0,1 bis 10 µg/l,
- Dexametason in einer Konzentration von 1 bis 10 mg/l und
- anorganische Salze.

10. Zusammensetzung nach Anspruch 9, wobei die anorganischen Salze ausgewählt sind aus der Gruppe bestehend aus einbasischem wasserfreiem Kaliumphosphat in einer Konzentration von 100 bis 500 mg/l und zweibasischem wasserfreiem Kaliumphosphat in einer Konzentration von 1,000 bis 2,500 mg/l.

11. Zusammensetzung nach Anspruch 1, wobei die Zusammensetzung ein spezialisiertes Zellkulturmedium ist, das angepasst ist zur Züchtung von Stammzellen und ferner umfasst:
- humanen rekombinanten Leukämieinhibitorfaktor in einer Konzentration von 100 bis 10.000 UI/l,
- Nucleoside,
- Adenosintriphosphat in einer Konzentration von 1 bis 10 mg/l,
- Thymidin in einer Konzentration von 5 bis 10 mg/l,
- Guanosin in einer Konzentration von 10 bis 50 mg/l,
- Uridin in einer Konzentration von 10 bis 50 mg/l,
- 2-b-Mercaptoethanol in einer Konzentration von 10 bis 100 µg/l, und
- Forscholin in einer Konzentration von 0,1 bis 10 mg/l.

12. Zusammensetzung nach Anspruch 1, die ferner 10%-iges DMSO (Dimethylsulfoxid) umfasst, um die Lebensfähigkeit der Zellen während der Lagerung bei niedrigen Temperaturen zu erhalten.

## Revendications

1. Composition pour l'établissement, le maintien, la multiplication et la congélation de lignées cellulaires, qui comprend :
• un concentré de plaquettes en une proportion de 1 à 30 %,
• des facteurs de croissance tels que le IGF-1, le IGF-11, le TGF-β, et
• les suppléments suivants :
- de l'adénosine-triphosphate en une quantité de 1 à 10 mg/l,
- du bicarbonate de sodium en une quantité de 1,2 à 5 g/l,
- de la cyticholine en une quantité de 10 à 100 mg/l,
- de l'éthanolamine en une quantité de 10 à 50 mg/l,
- de la phosphoéthanolamine en une quantité de 5 à 25 µg/l,
- du glucagon en une quantité de 1 à 5 mg/l,
- de la 1-glutamine en une quantité de 2 à 10 mM,
- de l'héparine sodique en une quantité de 10 000 à 50 000 UI/l,
- de l'hydrocortisone en une quantité de 10 à 100 mg/l,
- de l'insuline humaine recombinante en une quantité de 100 à 1000 UI/l,
- de la lévothyroxine en une quantité de 50 à 200 µg/l,
- de l'acide linoléique en une quantité de 10 à 50 µg/l,
- de l'acide oléique en une quantité de 10 à 50 µg/l,
- du pyruvate de sodium en une quantité de 50 à 150 mg/l,
- du séléniate de sodium en une quantité de 10 à 50 mg/l,
- de la toxine cholérique en une quantité de 0,1 à 1 mg/l,
- des antibiotiques,
- de la choline en une quantité de 1 à 30 mg/l,
- de l'acide folique en une quantité de 1 à 10 mg/l,
- du myo-inositol en une quantité de 1 à 40 mg/l,
- du niacynamide en une quantité de 1 à 10 mg/l,
- de l'acide p-aminobenzoïque en une quantité de 1 à 10 mg/l,
- de l'acide D-pantothénique en une quantité de 1 à 15 mg/l,
- de la pyridoxine en une quantité de 1 à 10 mg/l,
- de la riboflavine en une quantité de 2 à 20 mg/l,
- de la thiamine en une quantité de 1 à 5 mg/l,
- de la vitamine B12 en une quantité de 1 à 10 µg/l, et
- des aminoacides.

2. Composition suivant la revendication 1, dans laquelle les antibiotiques sont choisis dans le groupe consistant en :
- de la pénicilline à une concentration de 100 000 UI/l,
- de la streptomycine à une concentration de 100 µg/l, et
- de l'amphotéricine B à une concentration de 2,5 µg/l.

3. Composition suivant la revendication 1, dans laquelle les aminoacides sont choisis dans le groupe consistant en:
- de la I-histidine en une quantité de 1 à 20 mg/l,
- de la I-isoleucine en une quantité de 4 à 50 mg/l,
- de la I-méthionine en une quantité de 1 à 25 mg/l,
- de la I-phénylalanine en une quantité de 2 à 20 mg/l,
- du I-tryptophanne en une quantité de 1 à 5 mg/l, et
- de la I-tyrosine en une quantité de 2 à 10 mg/l.

4. Composition suivant la revendication 1, qui comprend en outre de l'albumine humaine présente à une concentration de 100 à 1000 mg/l et de la transferrine humaine présente à une concentration de 10 à 50 mg/l.

5. Composition suivant la revendication 1, dans laquelle les lignées cellulaires sont des lignées de cellules humaines et cellules animales.

6. Composition suivant la revendication 1, dans laquelle les cellules sont choisies dans le groupe consistant en des fibroblastes, des hépatocytes, des cellules épithéliales pigmentées rétiniennes et des cellules de lignées tumorales.

7. Composition suivant la revendication 1, ladite composition étant un milieu spécialisé de cultures cellulaires adapté à la culture d'adipocytes et comprenant en outre :
- de la biotine à une concentration de 1 à 10 mg/l, et
- de la dexaméthasone à une concentration de 1 à 10 mg/l.

8. Composition suivant la revendication 1, ladite composition étant un milieu spécialisé de cultures cellulaires adapté à la culture des mélanocytes et comprenant en outre :
- du facteur de croissance des fibroblastes basique (bFGF) à une concentration de 10 à 1000 µg/l, et
- de la théophylline à une concentration de 1 à 100 mg/l.

9. Composition suivant la revendication 1, ladite composition étant un milieu spécialisé de cultures cellulaires adapté à la culture des chondrocytes et des ostéoblastes et comprenant en outre :
- de l'acide I-ascorbique à une concentration de 20 à 100 mg/l,
- de la calcitonine humaine recombinante à une concentration de 100 à 10 000 UI/l,
- du calcitriol à une concentration de 0,1 à 10 µg/l,
- de la dexaméthasone à une concentration de 1 à 10 mg/l et
- des sels inorganiques.

10. Composition suivant la revendication 7, dans laquelle les sels inorganiques sont choisis dans le groupe consistant en le phosphate monopotassique anhydre à une concentration de 100 à 500 mg/l et le phosphate dipotassique anhydre à une concentration de 1000 à 2500 mg/l.

11. Composition suivant la revendication 1, ladite composition étant un milieu spécialisé de cultures cellulaires adapté à la culture des cellules souches et comprenant en outre :
- du facteur inhibiteur de leucémie humain recombinant à une concentration de 100 à 10 000 UI/l,
- des nucléosides,
- de l'adénosine-triphosphate à une concentration de 1 à 10 mg/l,
- de la thymidine à une concentration de 5 à 10 mg/l,
- de la guanosine à une concentration de 10 à 50 mg/l,
- de l'uridine à une concentration de 10 à 50 mg/l,
- du 2-β-mercaptoéthanol à une concentration de 10 à 100 µg/l, et
- de la forskoline à une concentration de 0,1 à 10 mg/l.

12. Composition suivant la revendication 1, qui comprend en outre du DMSO (diméthylsulfoxyde) en une teneur de 10 % afin de conserver la viabilité des cellules au cours du stockage à basse température.
